# FASCICULE DE BREVET EUROPEEN

(11) **EP 2 343 041 B1**
(45) Date de publication et mention de la délivrance du brevet: **19.07.2017**
(21) Numéro de dépôt: 10195236.4
(22) Date de dépôt: 15.12.2010
(51) Int. Cl.: A61K 8/39, A61K 8/42, A61K 8/46, A61K 8/58, A61K 8/73, A61K 8/86, A61K 8/87, A61Q 5/02, A61Q 5/12

(54) **Composition cosmétique comprenant au moins un composé organique du silicium, au moins un tensioactif anionique et au moins un agent épaississant non ionique ainsi qu'un procédé mettant en oeuvre la composition**
Kosmetische Zusammensetzung, die mindestens eine organische Siliziumverbindung, mindestens ein anionisches Tensid und mindestens ein nicht ionisches Verdickungsmittel umfasst, sowie ein Verfahren zum Einsatz dieser Zusammensetzung
Cosmetic composition containing at least one organic silicon compound, at least one anionic surface-active agent and at least one non-ionic thickener, as well as a method implementing the composition

(30) Priorité: 23.12.2009 FR 0959497
(43) Date de publication de la demande: 13.07.2011
(73) Titulaire: L'Oréal, 75008 Paris (FR)
(72) Inventeur: Viravau, Valérie, 75001, Paris (FR); Aires, Carine, 75014, Paris (FR)
(74) Mandataire: Dossmann, Gérard

(56) Documents cités:
- FR-A1- 2 789 896
- FR-A1- 2 930 439

## Description

La présente invention concerne une composition cosmétique destinée au nettoyage et au conditionnement des fibres kératiniques, en particulier des fibres kératiniques humaines telles que les cheveux, comprenant, dans un milieu cosmétiquement acceptable, un ou plusieurs composés organiques du silicium, un ou plusieurs tensioactifs anioniques, un ou plusieurs agents épaississants non ioniques, ladite composition présentant un rapport pondéral particulier entre ledit ou lesdits composés organiques du silicium et ledit ou lesdits tensioactifs anioniques.

La présente invention concerne également un procédé de traitement cosmétique des fibres kératiniques ainsi qu'une utilisation mettant en oeuvre ladite composition cosmétique.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base présentent certes un bon pouvoir lavant mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (c'est-à-dire les cheveux qui se trouvent généralement abîmés ou fragilisés par l'action des agents atmosphériques extérieurs tels que la lumière et les intempéries, et/ou des traitements mécaniques ou chimiques tels que le brossage, le peignage, les teintures, les décolorations, les permanentes et/ou les défrisages), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents de conditionnement peuvent bien entendu également améliorer le comportement cosmétique des cheveux naturels.

Dans ce but, on a déjà proposé d'utiliser des composés organiques cosmétiquement actifs tels que des polymères cationiques et des silicones en tant qu'agents de conditionnement, dans des compositions cosmétiques détergentes tels que des shampooings, pour conférer aux cheveux des propriétés cosmétiques satisfaisantes, en particulier de la brillance, de la douceur, de la souplesse, de la légèreté, un toucher naturel ainsi qu'une aptitude au démêlage améliorée.

Cependant, l'emploi de ces composés dans des compositions cosmétiques de lavage et de conditionnement ne procure pas aux cheveux des propriétés coiffantes satisfaisantes et durables. En effet, ces compositions procurent généralement des effets coiffants, tels que des effets de maintien, de corps et/ou de discipline des cheveux, qui restent souvent insuffisants et qui ont tendance à s'estomper après un lavage des cheveux avec un shampooing classique.

Or on a constaté que les consommateurs sont de plus en plus à la recherche de compositions de lavage qui soient non seulement capables de conditionner les cheveux de manière convenable mais également capables de procurer des effets coiffants satisfaisants et durables.

Ainsi des compositions destinées au lavage et au conditionnement des cheveux comprenant des composés organiques du silicium, tel que le 3-aminopropyltriéthoxysilane, ont été développées afin de pouvoir répondre à ces exigences. Ces compositions de lavage et de soin permettent de conditionner les cheveux, notamment en leur apportant un toucher doux satisfaisant, tout en conférant des effets coiffants marqués et durables.

De plus, ces compositions se sont révélées particulièrement avantageuses car elles permettent de faciliter la mise en forme des cheveux fins et de conférer des effets coiffants intéressants aux cheveux bouclés ou frisés, notamment en améliorant le dessin et le contrôle des boucles.

Toutefois, les compositions de lavage comprenant de tels composés organiques du silicium présentent généralement l'inconvénient d'évoluer sensiblement au cours du temps dans des conditions normales de stockage en fonction de la température, notamment au niveau de leur viscosité et de leur aspect visuel. En d'autres termes, ces compositions ne s'avèrent pas stables ce qui se traduit le plus souvent par un aspect visuel trouble ainsi que par une texture non satisfaisante au stockage.

En effet, il a été constaté que les composés organiques du silicium, tel que le 3-aminopropyltriéthoxysilane, n'étaient pas compatibles chimiquement avec la totalité des tensioactifs, notamment les tensioactifs anioniques, qui peuvent être présents dans les compositions de lavage, ce qui engendre les problèmes de stabilité rencontrés.

Par ailleurs, on a observé que l'introduction de certains composés organiques du silicium, en particulier les dérivés aminés, tels que le 3-aminopropyltriéthoxysilane, dans des compositions de lavage, qui présentent généralement un pH allant de 4 à 7, engendre également des problèmes de stabilité du fait du caractère alcalin de ces composés.

Il existe donc un réel besoin de mettre au point des compositions cosmétiques destinées au nettoyage et au conditionnement des fibres kératiniques contenant des composés organiques du silicium ne présentant pas l'ensemble des inconvénients décrits ci-dessus, c'est-à-dire qui sont stables dans le temps et qui permettent de conditionner les cheveux de manière satisfaisante tout en apportant des effets coiffants puissants durables, notamment en termes de masse, de corps, de texturisation des cheveux.

La demanderesse a découvert, de façon surprenante, qu'il était possible de formuler des compositions détergentes et conditionnantes des fibres kératiniques, ayant les propriétés recherchées, comprenant un ou plusieurs composés organiques du silicium tels que définis ci-après, un ou plusieurs tensioactifs anioniques, un ou plusieurs agents épaississants non ioniques, ladite composition présentant un rapport pondéral entre ledit ou lesdits composés organiques du silicium et ledit ou lesdits tensioactifs anioniques au moins égal à 5.10⁻⁴.

En effet, il a été constaté que l'utilisation d'un ou plusieurs agents épaississants non ioniques dans des compositions cosmétiques comprenant un ou plusieurs composés organiques du silicium tels que définis ci-après, un ou plusieurs tensioactifs anioniques et présentant un rapport pondéral entre ledit ou lesdits composés organiques du silicium et ledit ou lesdits tensioactifs anioniques qui est au moins égal à 5.10⁻⁴ permettait de rendre ces compositions stables au stockage aussi bien à température ambiante (20-25°C) qu'à 45°C, notamment au niveau de leur aspect visuel et de leur viscosité.

Par « stables » au sens de la présente invention, on entend que l'aspect visuel ainsi que la viscosité de ces compositions n'évoluent pas sensiblement au cours du temps dans des conditions standards de tests de stockage par exemple pendant les 2 mois à température ambiante (20°C-25°C), et/ou à 45°C et/ou à 4°C, qui suivent leur fabrication.

De plus, les compositions conformes à l'invention conduisent à un traitement satisfaisant des cheveux pouvant ainsi leur conférer un toucher doux satisfaisant, une aptitude au démêlage améliorée, de la douceur et de la souplesse.

Par ailleurs, les compositions conformes à la présente invention apportent des effets coiffants puissants, notamment en leur apportant de la masse, du corps et/ou de la discipline et ceci de manière durable.

De plus, les compositions selon l'invention permettent de faciliter la mise en forme des cheveux, en particulier des cheveux fins, et de conférer des effets coiffants améliorés aux cheveux bouclés, notamment en terme de dessin et de contrôle des boucles, et ceci de manière durable.

La présente invention concerne notamment une composition cosmétique pour le lavage et le conditionnement des fibres kératiniques, en particulier des fibres kératiniques humaines telles que les cheveux, comprenant dans un milieu cosmétiquement acceptable :
(i) de 0,01 à 5% en poids, par rapport au poids total de ladite composition, d'un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques, et
(iii) un ou plusieurs agents épaississants non ioniques, ladite composition cosmétique présentant un rapport pondéral entre ledit ou lesdits composés organiques du silicium et ledit ou lesdits tensioactifs anioniques au moins égal à 5.10⁻⁴.

La présente invention concerne également un procédé de traitement cosmétique des fibres kératiniques, en particulier de lavage et de conditionnement, comprenant l'application sur lesdites fibres de la composition selon l'invention.

Elle concerne aussi l'utilisation de la composition selon l'invention comme shampoing pour le lavage et le conditionnement des cheveux.

D'autres objets et caractéristiques, aspects et avantages de l'invention apparaîtront encore plus clairement à la lecture de la description et des exemples qui suivent.

Les composés organiques du silicium (i) utilisés dans la composition selon l'invention sont choisis parmi les organosilanes comprenant un atome de silicium et les organosiloxanes comportant deux ou trois atomes de silicium, de préférence deux atomes de silicium. Ils doivent en outre comporter une ou plusieurs fonctions chimiques basiques, et de préférence une seule fonction chimique basique. La fonction chimique basique peut correspondre à toute fonction conférant un caractère basique au composé de silicium et est de préférence une fonction amine telle qu'une fonction amine primaire, secondaire ou tertiaire. Les composés du silicium selon l'invention, peuvent comporter éventuellement d'autres fonctions, telles que, par exemple, une fonction acide ou une fonction halogène.

Le ou les composés organiques du silicium (i) utilisés dans la composition selon invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les organosilanes utilisées dans la composition selon l'invention sont choisis parmi les composés de formule (I) : 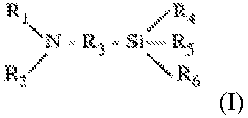 dans laquelle :
R₄ représente un halogène, un groupe OR' ou R'₁ ;
R₅ représente un halogène, un groupe OR" ou R'₂ ;
R₆ représente un halogène, un groupe OR"' ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R',
R" et R"' étant différents de l'hydrogène.

De préférence, les groupes R₁, R₂, R', R'₁, R'₂, R'₃, R" et R'" sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

Selon un autre mode de réalisation particulier, le ou les organosiloxanes utilisées dans la composition selon l'invention sont choisis parmi les composés de formule (II) : 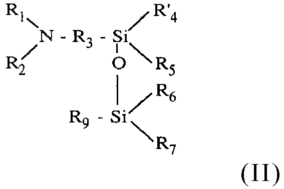 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR"', OR₁₀ ou OR₈.

De préférence, les groupes R"₁, R"₂, R₈ ou R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

En particulier, l'atome d'halogène est un atome de chlore.

Le ou les composés organiques du silicium utilisés dans la composition selon invention sont de préférence des organosilanes choisis parmi les composés de formule (III) : 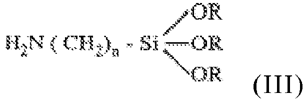 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium (i) présent dans la composition selon l'invention est le 3-aminopropyltriéthoxysilane.

Le ou les composés organiques du silicium peuvent être présents dans la composition selon l'invention dans une teneur allant, de préférence, de 0,01 à 4,5 % en poids, de préférence dans une teneur en poids allant de 0,1 à 2,5% en poids, et plus préférentiellement dans une teneur en poids allant de 0,2 à 2% en poids, par rapport au poids total de la composition.

Comme indiqué précédemment, la composition cosmétique selon la présente invention contient en outre un ou plusieurs tensioactifs anioniques (ii).

Les tensioactifs anioniques (ii) utilisés dans les compositions cosmétiques de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50, mieux de 2 à 10 et encore mieux de 2 à 5 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

De préférence, le ou les tensioactifs anioniques utilisés dans la composition cosmétique selon l'invention sont choisis parmi les alkyléthersulfates de préférence en C₁₂-C₁₄, et plus particulièrement comprenant de 2 à 3 moles d'oxyde d'éthylène et N-acyl taurates.

Les tensioactifs anioniques peuvent être présents dans une teneur allant de 1 à 25% en poids, de préférence dans une teneur allant de 3 à 20% en poids, et encore plus préférentiellement dans une teneur allant de 5 à 15% en poids, par rapport au poids total de la composition cosmétique selon l'invention.

Comme indiqué précédemment, la composition cosmétique selon la présente invention contient en outre un ou plusieurs agents épaississants non ioniques (iii).

Par épaississant, on entend au sens de la présente invention, tout composé dont la présence augmente la viscosité de la composition dans laquelle il est introduit d'au moins 25 cps, de préférence 50 cps à 25°C et à un taux de cisaillement de 1s⁻¹.

Le ou les agents épaississants non ioniques (iii) peuvent être choisis parmi les amides d'acide gras, les esters d'acides gras oxyalkylénés, les polymères épaississants non ioniques ou leurs mélanges.

Par « amide d'acide gras », on entend, au sens de la présente invention, un amide comportant dans sa structure au moins une chaîne hydrocarbonée comprenant au moins 6 atomes de carbone.

Les amides d'acides gras sont plus particulièrement choisis parmi les composés dérivant d'un amide d'alcanolamine et d'un acide gras en C₈-C₃₀, saturé ou insaturé, linéaire ou ramifié, l'alcanolamine et/ou l'acide gras étant éventuellement oxyalkyléné et plus particulièrement oxyéthyléné avec 1 à 50 moles d'oxyde d'éthylène.

De préférence, ils sont choisis parmi les amides d'une alcanolamine en C₂-C₁₀ et d'un acide gras en C₁₄-C₃₀, et encore plus préférentiellement parmi les amides d'une alcanolamine en C₂-C₁₀ et d'un acidegras en C₁₄-C₂₂.

Avantageusement, l'amide d'acide gras est choisi parmi :
- le monoisopropanolamide d'acide de coprah tel que l'amide commercialisé sous la dénomination commerciale EMPILAN CLS par la société HUNSTMAN,
- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN ® MM par la société COGNIS,
- le diéthanolamide d'acides gras de soja, tel que l'amide commercialisé sous la dénomination commerciale COMPERLAN ® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE ® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID ® 972 par la société ICI/UNIQEMA,
- le monoéthanolamide d'acide béhénique, tel que l'amide commercialisée sous la dénomination commerciale INCROMIDE ® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS,
- l'amide à 4 moles d'oxyde d'éthylène d'acide gras de colza tel que commercialisé sous l'appellation AMIDET N par la société KAO,

Le ou les agents épaississants non ioniques peuvent être choisis parmi les dérivés oxyalkylénés d'esters d'acide gras ou d'éthers d'alcools gras.

Comme dérivés oxyalkylénés d'esters d'acides gras, on peut notamment citer les dérivés alkyl ou acyl éthoxylés de polyols, qui peuvent être en particulier des dérivés oxyéthylénés d'esters d'acide gras en C₆-C₃₀ ou d'éthers d'alcool gras en C₆-C₃₀, et de polyols tels que le glycérol, sorbitol, glucose, pentaerythritol, le polyéthylèneglycol, plus particulièrement de polyéthylèneglycol, ces dérivés oxyéthylénés comportant généralement de 50 à 500 groupements oxyéthylénés et de préférence de 100 à 300 groupes oxyéthylénés.

Comme composés de ce type, on peut citer par exemple, le stéarate d'éthylène glycol, le distéarate de polyéthylène glycol comportant 150 groupes oxyéthylénés (150 OE), le stéarate de glycéryle oxyéthyléné (200 OE) tel que le produit commercialisé sous la dénomination SIMULSOL220 TM ® par la société Seppic, le tétrastéarate de pentaérythrityle oxyéthyléné (150 OE) tel que le produit commercialisésous la dénomination CROTHIX ® par la société Croda, le di-oléate de méthylglucose oxyéthyléné (120 OE) tel que leproduit commercialisé sous la dénomination GLUCAMATE DOE-120 VEGETAL® par la société Amerchol, le triisostéarate de sorbitan oxyéthyléné (160 OE) tel que le produit commercialisé sous la dénomination RHEODOL TW IS399C par la société Kao Chemicals, l'oléate de propylène glycol oxyéthyléné (55 oxyde d'éthylène) tel que le produit commercialisé sous la référence ANTIL 141 LIQUID par la société EVONIK GOLDSCHMIDT et leurs mélanges.

Dans une variante de l'invention, les esters d'acide gras oxyéthylénés sont de formule (A) suivante :

R₁-CO(X)ₙ-(OCH₂CH₂)ₘ-O-(CO)ₚ-R₂ (A)

X désignant un radical alkylène C₁-C₄, linéaire ou ramifié, et de préférence le radical de formule suivante : 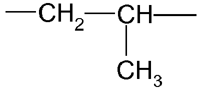
n désignant 0 ou 1,
p désignant 0 ou 1,
m allant de 50 à 200, et
R₁ désignant un radical alkyle ou alkényle, linéaire ou ramifié en C₉-C₂₉ et R₂ désignant un atome d'hydrogène ou un radical alkyle ou alkényle, linéaire ou ramifié en C₉-C₂₉.

Plus particulièrement, on préfère, parmi ces esters, le distéarate de polyéthylène glycol comportant 150 groupes oxyéthylénés (150 OE).

Les polymères épaississants de l'invention sont différents des amides et des esters déjà décrits ainsi que des produits ne résultant que de la simple condensation d'un oxyde d'alkylène sur un alcool, un ester, un amide.

Les polymères épaississants non ioniques peuvent être associatifs ou non.

Par « polymère épaississant non associatif », on entend selon l'invention, un polymère épaississant ne comportant pas à la fois au moins une chaîne grasse en C₈-C₃₀ et au moins un motif hydrophile.

Les polymères épaississants non ioniques non associatifs selon l'invention peuvent être d'origine naturelle ou synthétique. Ils sont notamment choisis parmi :
(i) les homopolymères et copolymères non-ioniques contenant des monomères à insaturation éthylénique de type ester et/ou amide,
(ii) les homo ou copolymères de vinylpyrrolidone,
(iii) les polysaccharides.

Parmi les homopolymères ou copolymères non-ioniques contenant des monomères à insaturation éthylénique de type ester et/ou amide, on peut citer les polyamides notamment les produits vendus sous les dénominations de : CYANAMER P250 par la société CYTEC (polyacrylamide); les copolymères méthacrylate de méthyle / diméthacrylate d'éthylèneglycol (PMMA MBX-8C par la société US COSMETICS) ; les copolymères méthacrylate de butyle / méthacrylate de méthyle (ACRYLOID B66 par la société RHOM & HAAS), les polyméthacrylate de méthyle (BPA 500 par la société KOBO);

Les homo ou copolymères de vinylpyrrolidone sont notamment choisis parmi les homopolymères de vinylpyrrolidone réticulés tels que le POLYMER ACP-10 commercialisé par ISP;

Les polysaccharides épaississant sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, les arabinogalactanes, les carraghénines, les agars, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar), et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

On utilisera de préférence, les amidons, les gommes de guar, les celluloses et leurs dérivés.

Les polysaccharides peuvent être modifiées ou non modifiées.

Les gommes de guar non modifiées sont par exemple les produits vendus sous la dénomination VIDOGUM GH 175 par la société UNIPECTINE, et sous les dénominations MEYPRO-GUAR 50 et JAGUAR C par la société RHODIA CHIMIE.

Les gommes de guar non-ioniques modifiées sont notamment modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE ou sous la dénomination GALACTASOL 4H4FD2 par la société AQUALON.

Parmi les celluloses, on utilise notamment les hydroxyéthyl celluloses, les hydroxypropylcelluloses. On peut citer les produits vendus sous les dénominations KLUCEL EF, KLUCEL H, KLUCEL LHF, KLUCEL MF, KLUCEL G, par la société AQUALON, le CELLOSIZE POLYMER PCG-10 par la société AMERCHOL.

Les polymères épaississants non ioniques associatifs selon l'invention peuvent être choisis parmi :
- (1) les celluloses modifiées par des groupements comportant au moins une chaîne grasse ; pour lesquelles peut citer à titre d'exemples :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse, tel que par exemple, le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemples :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse, tel que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tel que par exemple le copolymère méthacrylate de polyéthylèneglycol/méthacrylate de lauryle.
- (6) les polyuréthanes polyéthers comportant dans leur chaîne, à la fois des séquences hydrophiles, de nature le plus souvent polyoxyéthylénée, et des séquences hydrophobes qui peuvent être par exemple des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

En particulier, il est possible qu'une ou plusieurs chaînes pendantes soient prévues.

En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non ioniques comportent une liaison uréthanne entre les séquences hydrophiles, d'où l'origine du nom.

Par extension figurent aussi parmi les polyéthers polyuréthanes non ioniques à chaîne grasse, ceux dont les séquences hydrophiles sont liées aux séquences lipophiles par d'autres liaisons chimiques.

A titre d'exemples de polyéthers polyuréthanes non ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂₋ C₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

De préférence, les agents épaississants non ioniques sont choisis parmi les amides acides gras et les esters oxyéthylénés d'acides gras décrits ci-avant.

Le ou les agents épaississants non ioniques (iii) peuvent être présents dans une teneur allant de 0,1% à 20% en poids, de préférence dans une teneur allant de 0,1 à 10% en poids, mieux encore dans une teneur allant de 0,2 à 5% en poids, par rapport au poids total de la composition.

Comme indiqué précédemment, le rapport pondéral entre ledit ou lesdits composés organiques du silicium et ledit ou lesdits tensioactifs anioniques est égal à au moins 5.10⁻⁴.

De préférence, le rapport pondéral entre ledit ou lesdits composés organiques du silicium et ledit ou lesdits tensioactifs anioniques varie de 0,001 à 1 et varie, encore plus préférentiellement, de 0,01 à 0,8, mieux de 0,02 à 0,5.

La composition selon la présente invention peut comprendre également un ou plusieurs tensioactifs additionnels choisis parmi les tensioactifs amphotères et les tensioactifs non ioniques.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (IV) et (V) :

Rₐ-CONHCH₂CH₂-N(R_{b})(R_{c})(CH₂COO⁻) (IV)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (V)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,1 à 15% en poids, mieux encore de 0,5 à 10% en poids par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels varie de préférence de 0,01 à 20% en poids, mieux encore de 0,1 à 10 % en poids par rapport au poids total de la composition.

De préférence, la quantité totale en tensioactifs dans la composition cosmétique selon l'invention varie de 3 à 50% en poids, plus préférentiellement de 5 à 30% en poids, mieux de 8 à 20% en poids, par rapport au poids total de la composition cosmétique.

La composition cosmétique peut également comprendre un ou plusieurs polymères cationiques.

Au sens de la présente invention, l'expression "polymère cationique" désigne tout polymère contenant des groupements cationiques et/ou des groupements ionisables en groupements cationiques.

Les polymères cationiques éventuellement présents dans la composition selon l'invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VI), (VII), (VIII) ou (IX) suivantes: 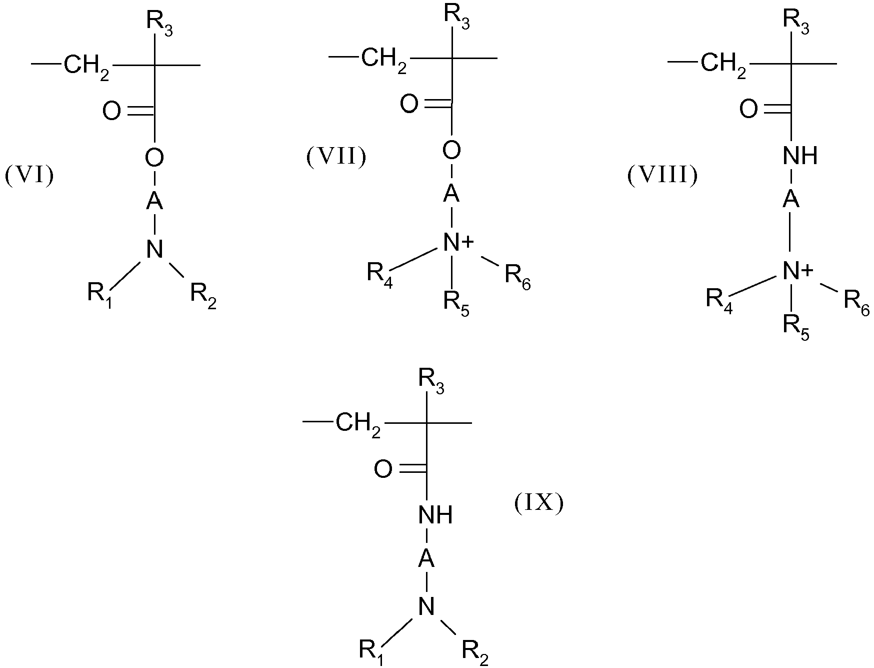 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   On peut citer en particulier l'homopolymère chlorure de méthacrylate d'éthyl triméthyl ammonium.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - les copolymères vinylpyrrolidone / méthacrylamide de diméthylaminopropyle quaternisés tel que le produit vendu sous la dénomination "GAFQUAT HS 100" par la société ISP,
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92" par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les dérivés d'éthers de cellulose comportant des groupements ammonium quaternaire décrits dans le brevet français 1 492 597, et en particulier les polymères commercialisés sous les dénominations "UCARE ˙˙POLYMER JR" (JR 400, JR 125, JR 30M) ou "LR" (LR 400, LR 30M) par la société ARMERCHOL. Ces polymères sont également définis dans le dictionnaire CTFA comme des ammoniums quaternaires d'hydroxyéthylcellulose ayant réagi avec un époxyde substitué par un groupement triméthylammonium.
(3) Les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les gommes de guar cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (X) ou (XI) : 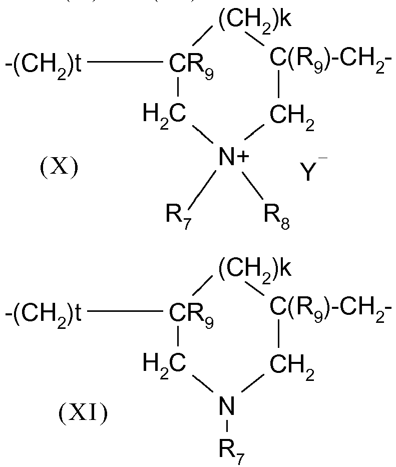 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faible masse moléculaire moyenne en poids) et les copolymères de chlorure de diallyldiméthylammonium et d'acrylamide commercialisés sous la dénomination "MERQUAT 550", "MERQUAT 7SPR".
(10) Le polymère de diammonium quaternaire contenant des motifs récurrents répondant à la formule : 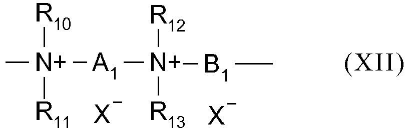 formule (XVI) dans laquelle :
   R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 8 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (XIII) suivante: 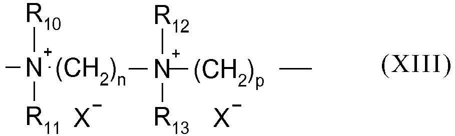 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 8 environ et, X⁻ est un anion dérivé d'un acide minéral ou organique. On peut citer en particulier le MEXOMERE PO commercialisé par la société CHIMEX.
(11)Les polyammoniums quaternaires constitués de motifs récurrents de formule (XIV) : 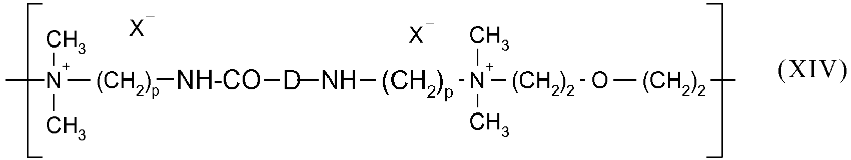
   dans laquelle p désigne un nombre entier variant de 1 à 6 environ, D peut être nul ou peut représenter un groupement -(CH₂)ᵣ - CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F. Ces polymères peuvent également comprendre d'autres monomères comme les halogénures de diallyldialkylammonium. On peut citer, en particulier, le produit commercialisé sous la dénomination Luviquat Sensation par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA, ou les polyamines de coprah oxyéthyléné (15 OE).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (2), (4), (9), (10) et (12).

De préférence, le ou les polymères cationiques sont choisis parmi les celluloses cationiques, les gommes de guar cationiques et les polymères quaternaires de vinylpyrrolidone et de vinyl imidazole éventuellement associé à d'autres monomères.

Plus préférentiellement, le ou les polymères cationiques sont choisis parmi les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyltriméthylammonium, de diméthyldiallyl ammonium, les gommes de guar cationiques le copolymère vinylpyrrolidone et de vinylimidazole et chlorure de diméthyldiallylammonium.

La teneur en polymère(s) cationique(s) dans la composition selon l'invention peut varier de 0,01 à 5% en poids par rapport au poids total de la composition, de préférence de 0,1 à 1% et plus préférentiellement de 0,15 à 0,5%.

La composition cosmétique selon la présente invention peut également contenir une ou plusieurs silicones, de préférence, aminées.

Par « silicone aminée », on entend, au sens de la présente invention, toute silicone comportant au moins une fonction amine primaire, secondaire ou tertiaire ou un groupement ammonium quaternaire.

Les silicones aminées éventuellement utilisées dans la composition cosmétique selon la présente invention sont choisies parmi :

### (a) les composés répondant à la formule (XV) suivante :

(R¹)ₐ(T)₃₋ₐ-Si[OSi(T)₂]ₙ-[OSi(T)_{b}(R¹)₂-_{b}]ₘ-OSi(T)₃₋ₐ-(R¹)a (XV)

dans laquelle,
T est un atome d'hydrogène, ou un radical phényle, hydroxyle (-OH), ou alkyle en C₁-C₈, et de préférence méthyle ou alcoxy en C₁-C₈, de préférence méthoxy,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R₁ est un radical monovalent de formule -C_{q}H_{2q}L dans laquelle q est un nombre de 2 à 8 et L est un groupement aminé éventuellement quaternisé choisi parmi les groupements :

   -N(R²)-CH₂-CH₂-N(R²)₂ ;

   -N(R²)₂ ; -N⁺(R²)₃ Q⁻ ;

   -N⁺(R²) (H)² Q⁻ ;

   -N⁺(R²)₂HQ⁻ ;

   -N(R²)-CH₂-CH₂-N⁺(R²)(H)₂Q-,

   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (XV) sont choisies parmi les composés correspondant à la formule suivante :

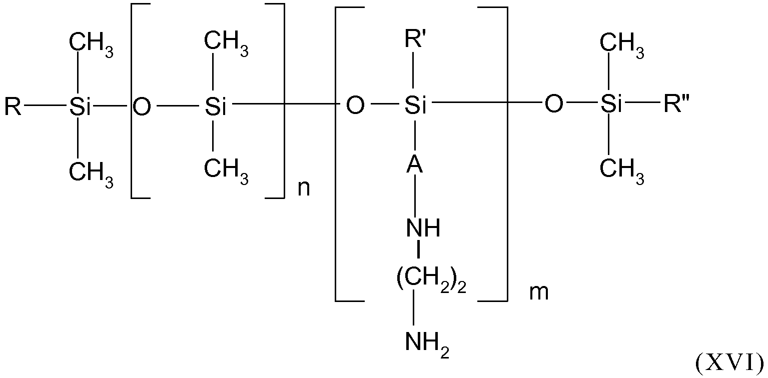

dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (XV) est en particulier le polymère dénommé dans le dictionnaire CTFA "triméthylsilylamodiméthicone", répondant à la formule (XVIII) suivante:

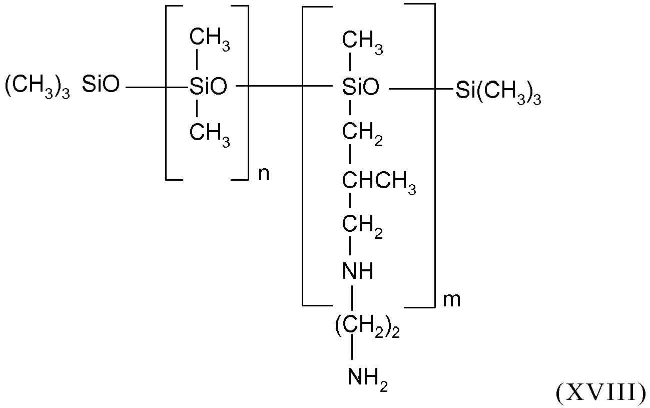

dans laquelle n et m ont les significations données ci-dessus conformément à la formule (XV).

De tels composés sont décrits par exemple dans EP 95238; un composé de formule (XVIII) est par exemple vendu sous la dénomination Q2-8220 par la société OSI.

### (b) les composés répondant à la formule (XIX) suivante :

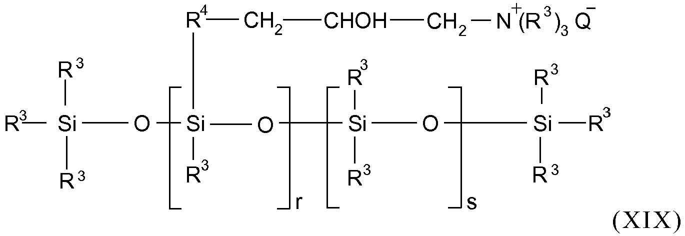

dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

### c) les silicones ammonium quaternaire de formule (XX) :

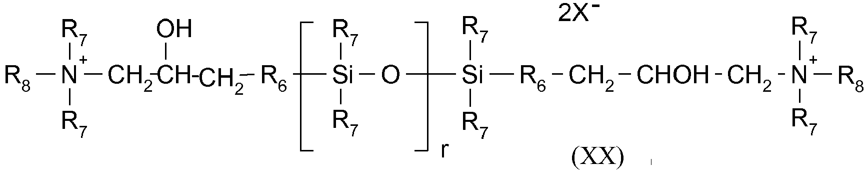

dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;

Ces silicones sont par exemple décrites dans la demande EP-A-0530974.

### d) les silicones aminées de formule (XXI) :

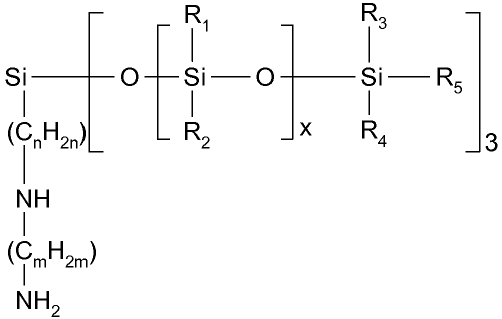

dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention varie généralement de 3 à 11 et de préférence de 5 à 10, mieux de 7 à 10.

La composition selon l'invention peut comprendre en outre un ou plusieurs additifs classiques bien connus dans la technique, tels que; des épaississants ou régulateurs de viscosité, naturels ou synthétiques autres que les épaississant non ioniques de l'invention ; des alcools gras en C₁₂-C₃₀ ; des céramides ; des esters gras huileux tels que le myristate d'isopropyle, le myristate de myristyle, le palmitate de cétyle et le stéarate de stéaryle ; des huiles minérales, végétales ou synthétiques telles que les α-oléfines ou l'huile de palme ; des vitamines ou provitamines ; des polymères amphotères ; des agents de stabilisation du pH, des conservateurs ; des silicones non aminées et des colorants.

Le ou les agents épaississants différents des agents épaississants non ioniques (iii) peuvent être choisis parmi les agents épaississants synthétiques tels que les homopolymères réticulés d'acide acrylique ou d'acide acrylamidopropanesulfonique, par exemple le Carbomer, les polymères associatifs anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

De préférence, les compositions cosmétiques de l'invention sont transparentes ou translucides, c'est-à-dire que ces compositions présentent une transmittance à 600 nanomètres supérieure à 85%, mieux supérieure à 90% et encore mieux supérieure à 94%.

Les compositions conformes à l'invention peuvent être utilisées comme shampooings pour le lavage et le conditionnement des cheveux et ils sont appliqués dans ce cas-là, de préférence, sur les cheveux humides dans des quantités efficaces pour les laver, et la mousse générée par massage ou friction avec les mains peut être ensuite éliminée après un éventuel temps de pause, par rinçage avec de l'eau, l'opération pouvant être répétée une ou plusieurs fois.

Un autre objet de l'invention est un procédé de traitement cosmétique des fibres kératiniques, telles que les cheveux, qui consiste à appliquer une quantité efficace d'une composition telle que décrite ci-dessus, sur lesdites fibres, et de préférence à rincer après un éventuel temps de pose.

En particulier, le procédé de traitement cosmétique des fibres kératiniques est un procédé de lavage et de conditionnement des fibres kératiniques, en particulier des cheveux.

Les exemples suivants servent à illustrer la présente invention.

### EXEMPLE

### Exemple 1 :

On prépare la composition (A) selon l'invention à partir des ingrédients indiqués dans le tableau ci-dessous dont les quantités sont exprimées en pour cent en poids en produit en l'état, par rapport au poids total de la composition.

| Composition | A (invention) |
|---|---|
| Acide lactique | 0,27 |
| Mélange de chloro-5-méthyl-2-iosthiazoline4-one-3 / méthylisothiazoline-4-one-3 / chlorure et nitrate de magnésium ⁽¹⁾ | 0,1 |
| Oléate de propylène glycol polyéthoxylé (55 OE) et de propylène glycol en solution hydroglycolique ⁽²⁾ | 0,6 |
| Hydroxyéthyl cellulose quaternisée par le chlorure de 2,3 époxypropyl triméthyl ammonium à 95,5% MA⁽³⁾ | 0,6 |
| Polydiméthyl siloxane à groupements ⁽⁴⁾ aminoéthyl aminoisobutyle et triméthylsiloxy | 1 |
| 3-aminopropyltriéthoxysilane ⁽⁵⁾ | 0,75 |
| Cocoyl bétaïne en solution aqueuse à 30% MA ⁽⁶⁾ | 17 |
| Alcool cétylique oxyéthyléné (20 OE) et oxypropyléné (5 OP) ⁽⁷⁾ | 0,5 |
| Acide lauryl éther carboxylique (4,5 OE) à 90% MA ⁽⁸⁾ | 1 |
| Monoisopropanolamide d'acides de coprah ⁽⁹⁾ | 0,85 |
| Lauryléther sulfate de sodium (2.2 OE) en solution aqueuse (70% M.A) ⁽¹⁰⁾ | 16 |
| Agent de mise au pH | qs pH = 9 |
| Parfum | 0,5 |
| Eau désionisée | qsp 100 g |

| | |
|---|---|
| ⁽¹⁾ vendu sous la dénomination commerciale KATHON CG par la société ROHM et HAAS ⁽²⁾ vendu sous la dénomination commerciale ANTIL 141 LIQUID par la société EVONIK GOLDSCHMIDT ⁽³⁾ vendu sous la dénomination commerciale POLYQUTA 400 KC par la société KCI ⁽⁴⁾ vendu sous la dénomination commerciale DC 8566 AMINOFLUID par la société DOW CORNING ⁽⁵⁾ vendu sous la dénomination commerciale XIAMETER OFS 6011 SILANE par la société DOW CORNING, ⁽⁶⁾ vendu sous la dénomination commerciale MIRATAINE BB/FLA par la société RHODIA, ⁽⁷⁾ vendu sous la dénomination commerciale PROCETYL AWS-LQ par la société CRODA, ⁽⁸⁾ vendu sous la dénomination commerciale AKYPO RLM 45 CA par la société KAO, ⁽⁹⁾ vendu sous la dénomination commerciale EMPILAN CIS par la société HUNTSMAN ⁽¹⁰⁾ vendu sous la dénomination Texapon AOS 225UP par la société COGNIS | |

On obtient une composition qui est limpide et stable dans le temps.

Appliquée en tant que shampooing, la composition (A) apporte des effets coiffants satisfaisants, en particulier cette composition confère aux cheveux de la masse, du volume ainsi qu'un toucher doux satisfaisant.

### Exemple 2 :

On prépare la composition (B) selon l'invention à partir des ingrédients indiqués dans le tableau ci-dessous dont les quantités sont exprimées en pour cent en poids en produit en l'état, par rapport au poids total de la composition.

| Composition | B |
|---|---|
| Acide lactique | 0,52 |
| Diméthylol-1,3-diméthyl-5,5 hydantoine en solution aqueuse ⁽¹⁾ | 0,25 |
| 3-aminopropyltriéthoxysilane ⁽²⁾ | 1,5 |
| Cocoyl bétaïne en solution aqueuse à 30% MA⁽³⁾ | 15 |
| Distéarate de polyéthylène glycol (150 OE) ⁽⁴⁾ | 1,1 |
| Cocoyl méthyl taurate de sodium en dispersion aqueuse à 30% MA⁽⁵⁾ | 20 |
| Agent de mise au pH | qs pH = 7 |
| Parfum | 0,5 |
| Eau désionisée | qsp 100 g |

| | |
|---|---|
| ⁽¹⁾ vendu sous la dénomination commerciale GLYDANT LTD par la société LONZA ⁽²⁾ vendu sous la dénomination commerciale XIAMETER OFS 6011 par la société DOW CORNING ⁽³⁾ vendu sous la dénomination commerciale MIRATAINE BB/FLA par la société RHODIA ⁽⁴⁾ vendu sous la dénomination commerciale KESSCO PEG 6000DS par la société HAMALACH CHEMICALS ARESE ⁽⁵⁾ vendu sous la dénomination commerciale HOSTAPON CT PATE par la société CLARIANT | |

On obtient également une composition qui est limpide et stable dans le temps.

Appliquée en tant qu'après shampooing, la composition (B) apporte des effets coiffants satisfaisants, en particulier cette composition confère aux cheveux de la masse, du volume ainsi qu'un toucher doux satisfaisant.

### Exemple 3 :

On prépare la composition (C) selon l'invention à partir des ingrédients indiqués dans le tableau ci-dessous dont les quantités sont exprimées en pour cent en poids en produit en l'état, par rapport au poids total de la composition.

| Composition | C |
|---|---|
| Chlorure de sodium | 1,44 |
| Acide lactique | 0,27 |
| Distéarate d'éthylèneglycol | 1,6 |
| Chlorure d'hydroxypropylguar triméthyl ammonium ⁽¹⁾ | 0,2 |
| Polymère carboxyvinylique ⁽²⁾ | 0,15 |
| 3-aminopropyltriéthoxysilane ⁽³⁾ | 0,75 |
| Hexylène glycol | 0,5 |
| Cocoyl bétaïne en solution aqueuse à 30%MA (4) | 6 |
| Monoisopropanolamide d'acides de coprah ⁽⁵⁾ | 0,7 |
| Lauryléther sulfate de sodium (2.2 OE) en solution aqueuse (70% M.A) ⁽⁶⁾ | 17,4 |
| Agent de mise au pH | qs pH = 9 |
| Parfum | 0,5 |
| Eau désionisée | qsp 100 g |

| | |
|---|---|
| ⁽¹⁾ vendu sous la dénomination commerciale JAGUAR C13S par la société RHODIA ⁽²⁾ vendu sous la dénomination commerciale CARBOPOL 980 par la société LUBRIZOL ⁽³⁾ vendu sous la dénomination commerciale XIAMETER OFS 6011 SILANE par la société DOW CORNING ⁽⁴⁾ vendu sous la dénomination commerciale MIRATAINE BB/FLA par la société RHODIA ⁽⁵⁾ vendu sous la dénomination commerciale EMPILAN CLS par la société HUNTSMAN ⁽⁶⁾ vendu sous la dénomination Texapon AOS 225UP par la société COGNIS | |

On obtient également une composition transparente et stable dans le temps.

Appliquée en tant que shampooing, la composition (C) confère aux cheveux de la masse, du volume ainsi qu'un toucher doux satisfaisant.

## Revendications

1. Composition cosmétique pour le lavage et le conditionnement des fibres kératiniques, en particulier des fibres kératiniques humaines telles que les cheveux, comprenant dans un milieu cosmétiquement acceptable :
(i) de 0,01 à 5% en poids, par rapport au poids total de ladite composition, d'un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques,
(iii) un ou plusieurs agents épaississants non ioniques,
ladite composition cosmétique présentant un rapport pondéral entre ledit ou lesdits composés organiques du silicium et ledit ou lesdits tensioactifs anioniques au moins égal à 5.10⁻⁴.

2. Composition cosmétique selon la revendication 1, **caractérisée en ce que** les fonctions chimiques basiques du composé organique du silicium sont choisies parmi les amines primaires, secondaires ou tertiaires.

3. Composition cosmétique selon la revendication 1 ou 2, **caractérisée en ce que** les groupes hydrolysables sont choisis parmi les groupes alcoxy, aryloxy et halogène.

4. Composition cosmétique selon l'une quelconque des revendications 1 à 3, **caractérisée en ce que** le ou les composés organiques du silicium sont choisis parmi les composés de formule (I) : 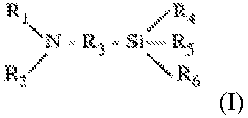 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R',
R" et R'" étant différents de l'hydrogène ; et 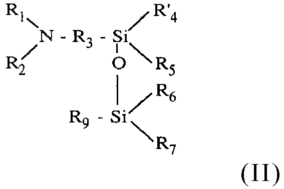 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR"', OR₁₀ ou OR₈.

5. Composition cosmétique selon la revendication 4, **caractérisée en ce que** les groupes R₁, R₂, R', R'₁, R'₂, R'₃, R", R"', R"₁, R"₂, R₈, R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C_{14-alkyle} de C₁ à C₈.

6. Composition cosmétique selon l'une quelconque des revendications précédentes, **caractérisée en ce que** le ou les composés organiques du silicium sont choisis parmi les composés de formule (III) : 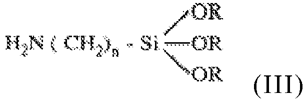 dans laquelle les radicaux R, identiques ou différents, sont choisis parmi les radicaux alkyle en C₁-C₆ et n est un nombre entier de 1 à 6, de préférence de 2 à 4.

7. Composition cosmétique selon l'une quelconque des revendications précédentes, **caractérisée en ce que** le ou les tensioactifs anioniques sont choisis parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

8. Composition cosmétique selon l'une quelconque des revendications précédentes, **caractérisée en ce que** le ou les agents épaississants non ioniques sont choisis parmi les amides d'acides gras, les esters oxyalkylénés d'acide gras, les polymères épaississants non ioniques ou leurs mélanges.

9. Composition cosmétique selon la revendication 8, **caractérisée en ce que** le ou les amides d'acides gras sont choisis parmi les amides d'une alcanolamine en C₂-C₁₀ et d'un acide gras en C₁₄-C₃₀, l'alcanolamine et/ou l'acide gras étant éventuellement oxyalkyléné, et encore plus préférentiellement parmi les amides d'une alcanolamine en C₂-C₁₀ et d'un acide gras en C₁₄-C₂₂.

10. Composition cosmétique selon la revendication 8, **caractérisée en ce que** le ou les polymères épaississants non ioniques sont choisis parmi les polymères non associatifs, et en particulier parmi les polysaccharides.

11. Composition cosmétique selon l'une quelconque des revendications 1 à 8, **caractérisée en ce que** le ou les polymères épaississants sont choisis parmi les polymères associatifs.

12. Composition cosmétique selon l'une quelconque des revendications précédentes, **caractérisée en ce que** le rapport pondéral entre ledit ou lesdits composés organiques du silicium et ledit ou lesdits tensioactifs anioniques varie de 0,001 à 1, encore plus préférentiellement de 0,01 à 0,8 et mieux de 0,002 à 0,5.

13. Composition cosmétique selon l'une quelconque des revendications précédentes, **caractérisée en ce qu'**elle comprend en outre un ou plusieurs tensioactifs additionnels choisis parmi les tensioactifs amphotères et les tensioactifs non ioniques.

14. Procédé de traitement cosmétique des fibres kératiniques, en particulier des fibres kératiniques humaines telles que les cheveux, **caractérisé en ce que** l'on applique la composition cosmétique telle que définie selon l'une quelconque des revendications précédentes, sur lesdites fibres, et **en ce que** l'on rince après un éventuel temps de pose.

15. Utilisation d'une composition selon l'une quelconque des revendications 1 à 13, pour le nettoyage et le conditionnement des fibres kératiniques.

## Patentansprüche

1. Kosmetische Zusammensetzung zur Reinigung und Konditionierung von Keratinfasern, insbesondere menschlichen Keratinfasern wie den Haaren, die in einem kosmetisch unbedenklichen Medium Folgendes umfasst:
(i) 0,01 bis 5 Gew.-%, bezogen auf das Gesamtgewicht der Zusammensetzung, einer oder mehrerer organischer Siliciumverbindungen, die aus Silanen mit einem Siliciumatom und Siloxanen mit zwei oder drei Siliciumatomen ausgewählt sind, wobei die organischen Siliciumverbindungen außerdem eine oder mehrere basische chemische Funktionen und eine oder mehrere Hydroxylgruppen oder hydrolysierbare Gruppen pro Molekül enthalten;
(ii) ein oder mehrere anionische Tenside,
(iii) ein oder mehrere nichtionische Verdickungsmittel,
wobei die kosmetische Zusammensetzung ein Gewichtsverhältnis von organischer Siliciumverbindung bzw. organischen Siliciumverbindungen und anionischem Tensid bzw. anionischen Tensiden von mindestens 5.10⁻⁴ aufweist.

2. Kosmetische Zusammensetzung nach Anspruch 1, **dadurch gekennzeichnet, dass** die basischen chemischen Funktionen der organischen Siliciumverbindung aus primären, sekundären oder tertiären Aminen ausgewählt sind.

3. Kosmetische Zusammensetzung nach Anspruch 1 oder 2, **dadurch gekennzeichnet, dass** die hydrolysierbaren Gruppen aus Alkoxy-, Aryloxy- und Halogengruppen ausgewählt sind.

4. Kosmetische Zusammensetzung nach einem der Ansprüche 1 bis 3, **dadurch gekennzeichnet, dass** die organische Siliciumverbindung bzw. die organischen Siliciumverbindungen aus den Verbindungen der Formel (I): 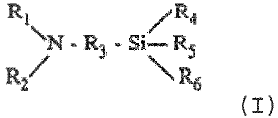 wobei:
R₄ für ein Halogen oder eine Gruppe OR' oder R'₁ steht;
R₅ für ein Halogen oder eine Gruppe OR'' oder R'₂ steht;
R₆ für ein Halogen oder eine Gruppe OR"' oder R'₃ steht;
R₁ R₂, R₃, R', R'', R''', R'₁, R'₂, R'₃ unabhängig voneinander für eine lineare oder verzweigte, gesättigte oder ungesättigte Kohlenwasserstoffgruppe, die gegebenenfalls zusätzliche chemische Gruppen trägt, stehen, wobei R₁, R₂, R', R" und
R"' außerdem Wasserstoff bedeuten können, und mindestens zwei der Gruppen R₄, R₅ und R₆ OR', OR" bzw. OR"' bedeuten, wobei mindestens zwei der Gruppen R', R" und R'" von Wasserstoff verschieden sind; und 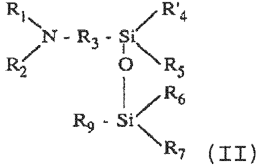 wobei:
R₁, R₂, R₃, R₅ und R₆ wie oben definiert sind;
R'₄ für ein Halogenatom oder eine Gruppe OR₁₁ steht;
R₇ für ein Halogenatom oder eine Gruppe OR₁₀ oder R''₁ steht;
R₉ für ein Halogenatom oder eine Gruppe OR₈, R''₂ oder R₃NR₁R₂ steht;
R''₁, R''₂, R₈, R₁₀ und R₁₁ für eine lineare oder verzweigte, gesättigte oder ungesättigte Kohlenwasserstoffgruppe, die gegebenenfalls zusätzliche chemische Gruppen trägt, stehen, wobei die Gruppen R₁₁, R₁₀ und R₈ außerdem für ein Wasserstoffatom stehen können; wobei mindestens eine der Gruppen R₆, R₇ und R₉ ein Halogenatom oder eine Gruppe OR''', OR₁₀ oder OR₈ bedeutet;
ausgewählt ist bzw. sind.

5. Kosmetische Zusammensetzung nach Anspruch 4, **dadurch gekennzeichnet, dass** die Gruppen R₁, R₂, R', R'₁, R'₂, R'₃, R'', R''', R''₁, R''₂, R₈, R₁₀ und R₁₁ aus C₁- bis C₁₂-Alkylresten, C₆- bis C₁₄-Arylresten, C₁-C₈-Alkyl-C₆-C₁₄-arylresten und C₆-C₁₄-Aryl-C₁-C₈-alkylresten ausgewählt sind.

6. Kosmetische Zusammensetzung nach einem der vorhergehenden Ansprüche, **dadurch gekennzeichnet, dass** die organische Siliciumverbindung bzw. die organischen Siliciumverbindungen aus Verbindungen der Formel (III): 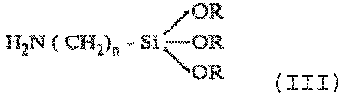 wobei die Reste R, die gleich oder verschieden sind, aus C₁- bis C₆-Alkylresten ausgewählt sind und n eine ganze Zahl von 1 bis 6, vorzugsweise von 2 bis 4, ist;
ausgewählt ist bzw. sind.

7. Kosmetische Zusammensetzung nach einem der vorhergehenden Ansprüche, **dadurch gekennzeichnet, dass** das anionische Tensid bzw. die anionischen Tenside aus Alkylsulfaten, Alkylethersulfaten und Alkylethercarboxylaten und Mischungen davon, insbesondere in Form von Alkalimetall-, Erdalkalimetall-, Ammonium-, Amin- oder Aminoalkoholsalzen ausgewählt ist bzw. sind.

8. Kosmetische Zusammensetzung nach einem der vorhergehenden Ansprüche, **dadurch gekennzeichnet, dass** das nichtionische Verdickungsmittel bzw. die nichtionischen Verdickungsmittel aus Fettsäureamiden, oxyalkylenierten Fettsäureestern, nichtionischen verdickenden Polymeren oder Mischungen davon ausgewählt ist bzw. sind.

9. Kosmetische Zusammensetzung nach Anspruch 8, **dadurch gekennzeichnet, dass** das Fettsäureamid bzw. die Fettsäureamide aus Amiden eines C₂-C₁₀-Alkanolamins und einer C₁₄-C₃₀-Fettsäure, wobei das Alkanolamin und/oder die Fettsäure gegebenenfalls oxyalkyleniert sind bzw. ist, und noch weiter bevorzugt aus Amiden eines C₂-C₁₀-Alkanolamins und einer C₁₄-C₂₂-Fettsäure ausgewählt ist bzw. sind.

10. Kosmetische Zusammensetzung nach Anspruch 8, **dadurch gekennzeichnet, dass** das nichtionische verdickende Polymer bzw. die nichtionischen verdickenden Polymere aus nicht assoziativen Polymeren und insbesondere Polysacchariden ausgewählt ist bzw. sind.

11. Kosmetische Zusammensetzung nach einem der Ansprüche 1 bis 8, **dadurch gekennzeichnet, dass** das verdickende Polymer bzw. die verdickenden Polymere aus assoziativen Polymeren ausgewählt sind.

12. Kosmetische Zusammensetzung nach einem der vorhergehenden Ansprüche, **dadurch gekennzeichnet, dass** das Gewichtsverhältnis von organischer Siliciumverbindung bzw. organischen Siliciumverbindungen und anionischem Tensid bzw. anionischen Tensiden im Bereich von 0,001 bis 1, noch weiter bevorzugt von 0,01 bis 0,8 und noch besser von 0,002 bis 0,5 liegt.

13. Kosmetische Zusammensetzung nach einem der vorhergehenden Ansprüche, **dadurch gekennzeichnet, dass** sie außerdem ein oder mehrere zusätzliche Tenside, die aus amphoteren Tensiden und nichtionischen Tensiden ausgewählt sind, umfasst.

14. Verfahren zur kosmetischen Behandlung von Keratinfasern, insbesondere menschlichen Keratinfasern wie den Haaren, **dadurch gekennzeichnet, dass** man die kosmetische Zusammensetzung gemäß einem der vorhergehenden Ansprüche auf die Fasern aufbringt und nach einer fakultativen Einwirkungszeit ausspült.

15. Verwendung einer Zusammensetzung nach einem der Ansprüche 1 bis 13 zur Reinigung und Konditionierung von Keratinfasern.

## Claims

1. Cosmetic composition for washing and conditioning keratin fibres, in particular human keratin fibres such as the hair, comprising, in a cosmetically acceptable medium:
(i) from 0.01% to 5% by weight, relative to the total weight of the said composition, of one or more organosilicon compounds chosen from silanes comprising one silicon atom and siloxanes comprising two or three silicon atoms, the said organosilicon compounds also comprising one or more basic chemical functions and one or more hydroxyl or hydrolysable groups per molecule;
(ii) one or more anionic surfactants, and
(iii) one or more nonionic thickeners,
the said cosmetic composition having a weight ratio between the said organosilicon compound(s) and the said anionic surfactant(s) at least equal to 5×10⁻⁴.

2. Cosmetic composition according to Claim 1, **characterized in that** the basic chemical functions of the organosilicon compound are chosen from primary, secondary and tertiary amines.

3. Cosmetic composition according to Claim 1 or 2, **characterized in that** the hydrolysable groups are chosen from alkoxy, aryloxy and halogen groups.

4. Cosmetic composition according to any one of Claims 1 to 3, **characterized in that** the organosilicon compounds(s) are chosen from the compounds of formula (I) : 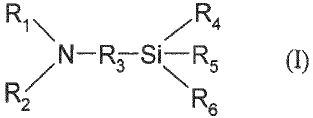 in which:
R₄ represents a halogen or a group OR' or R'₁;
R₅ represents a halogen or a group OR'' or R'₂;
R₆ represents a halogen or a group OR''' or R'₃;
R₁, R₂, R₃, R', R'', R''', R'₁, R'₂ and R'₃ represent, independently of each other, a saturated or unsaturated, linear or branched hydrocarbon-based group optionally bearing additional chemical groups, R₁, R₂, R', R'' and R''' also possibly denoting hydrogen, and at least two of the groups R₄, R₅ and R₆ respectively denoting OR', OR'' and OR''', at least two of the groups R', R'' and R''' being other than hydrogen; and 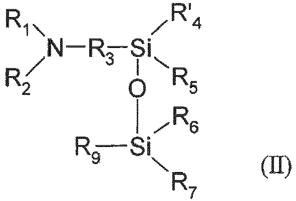 in which:
R₁, R₂, R₃, R₅ and R₆ are as defined previously;
R'₄ represents a halogen atom or a group OR₁₁;
R₇ represents a halogen atom or a group OR₁₀ or R''₁;
R₉ represents a halogen atom or a group OR₈, R''₂ or R₃NR₁R₂;
R''₁, R''₂, R₈, R₁₀ and R₁₁ represent a saturated or unsaturated, linear or branched hydrocarbon-based group, optionally bearing additional chemical groups, R₁₁, R₁₀ and R₈ also possibly representing a hydrogen atom; at least one of the groups R₆, R₇ and R₉ denoting a halogen atom or a group OR''', OR₁₀ or OR₈.

5. Cosmetic composition according to Claim 4, **characterized in that** the groups R₁, R₂, R', R'₁, R'₂, R'₃, R'', R''', R''₁, R''₂, R₈, R₁₀ and R₁₁ are chosen from C₁-C₁₂ alkyl, C₆-C₁₄ aryl, (C₁-C₈)alkyl(C₆-C₁₄)aryl and (C₆-C₁₄)aryl(C₁-C₈)alkyl radicals.

6. Cosmetic composition according to any one of the preceding claims, **characterized in that** the organosilicon compound(s) are chosen from the compounds of formula (III): 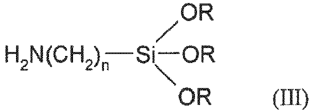 in which the radicals R, which may be identical or different, are chosen from C₁-C₆ alkyl radicals, and n is an integer from 1 to 6 and preferably from 2 to 4.

7. Cosmetic composition according to any one of the preceding claims, **characterized in that** the anionic surfactant(s) are chosen from alkyl sulfates, alkyl ether sulfates and alkyl ether carboxylates, and mixtures thereof, in particular in the form of alkali metal, alkaline-earth metal, ammonium, amine or amino alcohol salts.

8. Cosmetic composition according to any one of the preceding claims, **characterized in that** the nonionic thickeners(s) are chosen from fatty acid amides, oxyalkylenated fatty acid esters and nonionic thickening polymers, or mixtures thereof.

9. Cosmetic composition according to Claim 8, **characterized in that** the fatty acid amide(s) are chosen from amides of a C₂-C₁₀ alkanolamine and of a C₁₄-C₃₀ fatty acid, the alkanolamine and/or the fatty acid being optionally oxyalkylenated, and even more preferentially from amides of a C₂-C₁₀ alkanolamine and of a C₁₄-C₂₂ fatty acid.

10. Cosmetic composition according to Claim 8, **characterized in that** the nonionic thickening polymer(s) are chosen from non-associative polymers, and in particular from polysaccharides.

11. Cosmetic composition according to any one of Claims 1 to 8, **characterized in that** the thickening polymer(s) are chosen from associative polymers.

12. Cosmetic composition according to any one of the preceding claims, **characterized in that** the weight ratio between the said organosilicon compound(s) and the said anionic surfactant(s) ranges from 0.001 to 1, even more preferentially from 0.01 to 0.8 and better still from 0.002 to 0.5.

13. Cosmetic composition according to any one of the preceding claims, **characterized in that** it also comprises one or more additional surfactants chosen from amphoteric surfactants and nonionic surfactants.

14. Cosmetic process for treating keratin fibres, in particular human keratin fibres such as the hair, **characterized in that** the cosmetic composition as defined according to any one of the preceding claims is applied to the said fibres, and **in that** it is rinsed out after an optional leave-on time.

15. Use of a composition according to any one of Claims 1 to 13, for cleansing and conditioning keratin fibres.
